(19) Europäisches Patentamt
European Patent Office
Office européen des brevets

(11) EP 2 181 984 A1

(12) **EUROPEAN PATENT APPLICATION**
published in accordance with Art. 153(4) EPC

(43) Date of publication:
**05.05.2010 Bulletin 2010/18**

(21) Application number: **07855860.8**

(22) Date of filing: **28.12.2007**

(51) Int Cl.:
*C07C 323/58* (2006.01) *C07C 319/02* (2006.01)
*A61K 31/198* (2006.01) *A61P 39/02* (2006.01)
*A61P 43/00* (2006.01) *C07C 323/50* (2006.01)
*C07C 323/22* (2006.01) *C07C 323/00* (2006.01)
*C07C 319/00* (2006.01)

(86) International application number:
**PCT/CN2007/003859**

(87) International publication number:
**WO 2009/006773 (15.01.2009 Gazette 2009/03)**

(84) Designated Contracting States:
**AT BE BG CH CY CZ DE DK EE ES FI FR GB GR HU IE IS IT LI LT LU LV MC MT NL PL PT RO SE SI SK TR**
Designated Extension States:
**AL BA HR MK RS**

(30) Priority: **12.07.2007 CN 200710137428**

(71) Applicant: **Nanjing Sanhome Pharmaceutical Co., Ltd.**
**Nanjing,**
**Jiangsu 210038 (CN)**

(72) Inventors:
- **WANG, Yong**
  **Nanjing, Jiangsu 210038 (CN)**
- **ZHANG, Cang**
  **Nanjing, Jiangsu 210038 (CN)**
- **TENG, Zaijin**
  **Nanjing, Jiangsu 210038 (CN)**
- **ZHANG, Wenping**
  **Nanjing, Jiangsu 210038 (CN)**
- **QIAN, Jinye**
  **Nanjing, Jiangsu 210038 (CN)**

(74) Representative: **Wibbelmann, Jobst et al**
**Wuesthoff & Wuesthoff**
**Patent- und Rechtsanwälte**
**Schweigerstrasse 2**
**81541 München (DE)**

# (54) AN OPTICALLY ACTIVE N- (alpha-MERCAPTOPROPIONY) GLYCINE

(57) An optically active N-(α-mercaptopropionyl)glycine, i.e., R-(-)-N-(α-mercaptopropionyl)glycine or S-(-)-N-(α-mercaptopropionyl)glycine, a preparation method thereof, a pharmaceutical preparation containing the compound or a pharmaceutically acceptable salt or ester thereof, and use of the same in preparation of detoxification medicament for improving metabolism, are provided.

EP 2 181 984 A1

## Description

## BACKGROUND OF THE INVENTION

### Field of the Invention

The present invention relates to an optically active N-(α-mercaptopropionyl)glycine, preparation method thereof, use of the same in preparation of detoxification medicament for improving metabolism, and pharmaceutical preparation containing the same.

### Related Art

N-(α-mercaptopropionyl)glycine, generic name tiopronin, is used to treat, for example, acute and chronic hepatitis, and cirrhosis in clinics, with very excellent therapeutic use in treatment of liver diseases, and preparation method and pharmaceutical preparation thereof have been frequently disclosed. A tiopronin pharmaceutical preparation is disclosed in Chinese Patent Application No. CN02129300.7, and a process for synthesizing tiopronin is disclosed in Chinese Journal of Medicinal Chemistry (Vol. 7, No. 1, p. 55-56, Mar. 1997). However, there is not any report for the optical activity, i.e., levo and dextro isomers of N-(α-mercaptopropionyl)glycine. For the majority of the drugs, a single enantiomer has high therapeutic efficacy and low adverse effect, and thus is called as eutomer, while the inactive or low-active enantiomer is referred to as distomer; and in most cases, the distomer has no therapeutic efficacy, but can also partly neutralize the effect of the enantiomer, and even incur serious adverse effect. The enantiomeric drugs have very different pharmacologic activity and metabolic process in the body, and thus exhibit different biological activity and efficacy. In clinical application, due to the lack of awareness of the differences in pharmacodynamic and pharmacokinetic behaviors of the individual enantiomer of chiral drugs, the conclusions sometimes contradict the therapeutic efficacy or occurrence of adverse effect, and will even improperly direct the application of drugs in clinics. Therefore, researches on optical activities of drugs are very necessary.

## SUMMARY OF THE INVENTION

The present invention is directed to an optically active N-(α-mercaptopropionyl)glycine.
The present invention is further directed to a method for preparing the optically active N-(α-mercaptopropionyl) glycine.
The present invention is further directed to a use of the optically active N-(α-mercaptopropionyl)glycine in preparation of detoxification medicament for improving metabolism.
The present invention is further directed to a pharmaceutical preparation containing the optically active N-(α-mercaptopropionyl)glycine.
As embodied and broadly described herein, the present invention provides an optically active N-(α-mercaptopropionyl)glycine, which is levo or dextro N-(α-mercaptopropionyl)glycine.
The levo N-(α-mercaptopropionyl)glycine is R-(-)-N-(α-mercaptopropionyl)glycine

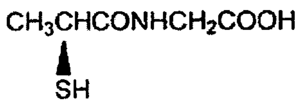

of R-configuration, or a pharmaceutically acceptable salt or ester thereof, in which the salt can be an amino acid salt or a metal salt.
The amino acid salt has a general structural formula below:

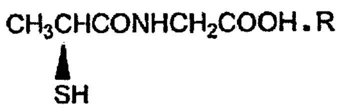
,

in which R can be an amino acid selected from, for example, arginine, lysine, glycine, aspartic acid, alanine, phenylalanine,

leucine, isoleucine, ornithine, cystine, cysteine, tyrosine, valine, serine, histidine, threonine, tryptoophan, methionine, methionine, proline, glutamic acid, and hydroxyproline.

The metal salt has a general structural formula below:

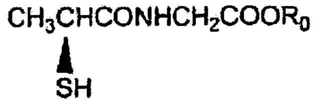

,

in which $R_0$ can be, for example, potassium or sodium.

The ester has a general structural formula below:

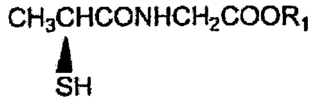

,

in which $R_1$ is a linear $C_1$-$C_5$ alkyl group.

The dextro N-(α-mercaptopropionyl)glycine is S-(+)-N-(α-mercaptopropionyl)glycine

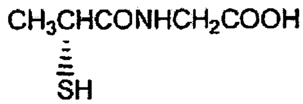

of S-configuration, or a pharmaceutically acceptable salt or ester thereof, in which the salt can be an amino acid salt or a metal salt.

The amino acid salt has a general structural formula below:

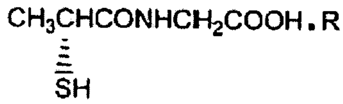

in which R can be an amino acid selected from, for example, arginine, lysine, glycine, aspartic acid, alanine, phenylalanine, leucine, isoleucine, ornithine, cystine, cysteine, tyrosine, valine, serine, histidine, threonine, tryptoophan, methionine, methionine, proline, glutamic acid, and hydroxyproline.

The metal salt has a general structural formula below:

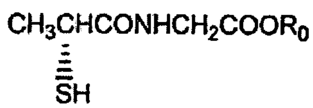

,

in which $R_0$ can be, for example, potassium or sodium.

The ester has a general structural formula below:

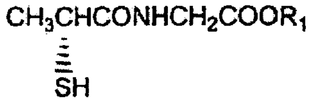

,

in which $R_1$ is a linear $C_1$-$C_5$ alkyl group.

The present invention also provides a method for preparing the optically active N-(α-mercaptopropionyl)glycine, which includes a method for preparing R-(-)-N-(α-mercaptopropionyl)glycine and a method for preparing S-(-)-N-(α-mercaptopropionyl)glycine.

I. The method for preparing R-(-)-N-(α-mercaptopropionyl)glycine includes:

1. reacting R-(+)-2-chloropropionic acid with thionyl chloride to get R-(+)-2-chloropropionyl chloride;

2. reacting R-(+)-2-chloropropionyl chloride with glycine under a weak basic condition to get R-(+)-2-chloropropionyl glycine; and

3. reacting sodium sulfide with sublimed sulfur to get sodium disulfide, which is then acidified by reacting with R-(+)-2-chloropropionyl glycine, to get R-(-)-N-(α-mercaptopropionyl)glycine;
where the resulting R-(-)-N-(α-mercaptopropionyl)glycine is further reacted with an acid, a base, or an alcohol to get a salt or an ester.

II. The method for preparing S-(+)-N-(α-mercaptopropionyl)glycine includes:

1. reacting S-(-)-2-chloropropionic acid with thionyl chloride to get S-(-)-2-chloropropionyl chloride;

2. reacting S-(-)-2-chloropropionyl chloride with glycine under a weak basic condition to get S-(-)-2-chloropropionyl glycine; and

3. reacting sodium sulfide with sublimed sulfur to get sodium disulfide, which is then acidified by reacting with S-(-)-2-chloropropionyl glycine, to get S-(+)-N-(α-mercaptopropionyl)glycine;
where the resulting S-(+)-N-(α-mercaptopropionyl)glycine is further reacted with an acid, a base, or an alcohol to get a salt or an ester.

The present invention also provides a use of the optically active N-(α-mercaptopropionyl)glycine, i.e., R-(-)-N-(α-mercaptopropionyl)glycine or S-(+)-N-(α-mercaptopropionyl)glycine, or a pharmaceutically acceptable salt or ester thereof in preparation of detoxification medicament for improving metabolism, and particularly a use in treatment of acute and chronic liver diseases and improvement of liver function, including protection of liver tissue cells, treatment of various hepatitis, for example, acute and chronic hepatitis, viral hepatitis, alcoholic hepatitis, drug induced hepatitis, and heavy metal toxic hepatitis, and treatment of fatty liver, acute and chronic liver injury, and cirrhosis; as well as in prevention and cure of peripheral blood leukopenia caused by chemoradiation therapy and accelerating of restoration of liver cells, to reduce the adverse effect of chemotherapy; in prevention and cure of early senile cataract and vitreous opacities; and in heavy metal detoxification.

It is found from pharmacodynamic experiment that both R-(-)-N-(α-mercaptopropionyl)glycine and S-(+)-N-(α-mercaptopropionyl)glycine have good protection effect for liver injury, which is better than that of N-(α-mercaptopropionyl) glycine.

It is found from pharmacokinetic experiment that, conversion of R-(-)-N-(α-mercaptopropionyl)glycine and S-(+)-N-(α-mercaptopropionyl)glycine to each other have not occurred in body. The specific experiment is a pharmacokinetic test in the body of a test animal, including determining the plasma drug concentration with high-performance liquid chromatography-mass spectrometry (HPLC-MS).

The process for treat a plasma sample to formulate an injected solution includes plasma acidification, extraction, and then derivatization, and is as follows:

1. plasma acidification, where the acid used for acidification can be, for example, hydrochloric acid, phosphoric acid, perchloric acid, or acetic acid, with hydrochloric acid being preferred, and hydrochloric acid of 1 mol/L being more preferred; and where the volume ratio of the acid to the plasma sample is (150 μl-250 μl):(2 ml-4 ml), with 200 μl:

3 ml being preferred;

2. extraction of the acidified plasma, where the organic solvent used for extraction can be, for example, ethyl acetate, chloroform, trichloromethane, diethyl ether, or n-hexane, with ethyl acetate being preferred; and

3. derivatization of the extract, where the useful derivatization reagent can be, for example, phenyl isothiocyanate and 2,3,4,6-tetra-O-acetyl-β-D-pyranoglucose isothiocyanate (GITC) solution, with GITC solution being preferred, GITC solution of 2 mg/ml being more preferred, and solution of 2 mg/ml GITC in tetrahydrofuran being most preferred; where the derivatization temperature is 15-45°C, with 25-35°C being preferred, and 30°C being more preferred; and where the derivatization time is 10-30 min, with 15-25 min being preferred, and 20 min being more preferred.

Chromatographic conditions include:

mobile phase A: methanol; mobile phase B: an aqueous solution, containing 0.05-0.20 mmol/L of sodium chloride and 5.0-6.0 mmol/L of formic acid, with 0.10 mmol/L of sodium chloride and 5.3 mmol/L of formic acid being preferred, where the ratio of A to B is (40-50:50-60, with 44:56 being preferred).

Mass spectrometric conditions include:

ionization mode: electrospray ionization; selective ion detection; curved desolvation line (CDL); temperature: 200°C-300°C, with 250°C being preferred; heating block temperature: 150°C-250°C, with 200°C being preferred; CDL voltage: 20V-30V, with 25V being preferred; detection voltage: +1.2 kV-+1.8 kV, with +1.50 kV being preferred; flow rate of atomizing gas: 1.2 L/min-1.8 L/min, with 1.5 L/min being preferred; flow rate of drying gas: 1.5 L/min-2.5 L/min, with 2.0 L/min being preferred; detected ion: derivative of test drug [M+Na]+(m/z): 575.20; internal standard: derivative of N-isobutanoyl-D-cysteine (NIDC) [M+Na]+(m/z): 603.05;
as long as the separation degree of the internal standard peak from the major peak meets the requirements of Chinese Pharmacopoeia.

The results indicate that, in spectrogram of R-(-)-N-(α-mercaptopropionyl)glycine at each time points after being administrated individually, the presence of S-(+)-N-(α-mercaptopropionyl)glycine is not obviously detected; likewise, the presence of R-(-)-N-(α-mercaptopropionyl)glycine is not obviously detected either after administrating S-(+)-N-(α-mercaptopropionyl)glycine, which suggest that conversions to each other have not occurred in body.

Therefore, it is practicable to make clinically useful pharmaceutical preparation from R-(-)-N-(α-mercaptopropionyl)glycine or S-(+)-N-(α-mercaptopropionyl)glycine.

In the present invention, the optically active N-(α-mercaptopropionyl)glycine is made into a clinically useful pharmaceutical preparation, which contains R-(-)-N-(α-mercaptopropionyl)glycine or S-(+)-N-(α-mercaptopropionyl)glycine, or a pharmaceutically acceptable salt or ester thereof as active ingredient, and a pharmaceutically acceptable adjuvant, and is an orally administrated preparation or an injection preparation.

The orally administrated preparation includes: a general oral preparation, for example, tablet, capsule, granule, chewable tablet, or effervescent tablet; a rapid release preparation, for example, dispersible tablet, or orally disintegrating tablet; or a slow release preparation, for example, slow release tablet, or slow release pellet. The injection preparation includes, for example, injectable solution, concentrated solution for injection, or sterile powder for injection.

The adjuvant in the oral pharmaceutical preparation includes a filler, a binder, or a disintegrant, in which the weight contents of the filler and the disintegrant are 10-60%, and 2-30% respectively; and a glidant , a lubricant, and a surfactant can optionally exist, in which the weight contents of the glidant , the lubricant, and the surfactant are 0.1-5%, 0.1-5%, and 0.005-1% respectively. The filler can be starch, pregelatinized starch, carboxymethyl starch, microcrystalline cellulose, lactose, dextrin, sucrose, glucose, mannitol, sorbitol, calcium sulfate dihydrate, dibasic calcium phosphate, tribasic calcium phosphate, or calcium carbonate. The binder can be corn starch, pregelatinized corn starch, pregelatinized starch, gelatine, sucrose, arabic gum, povidone, methylcellulose of various viscosities, sodium carboxymethyl cellulose of low viscosity, ethylcellulose of various viscosities, polyvinyl alcohol of various viscosities, polyethylene glycol 6000, or solution of hydroxypropylmethyl cellulose in water or an alcohol. The disintegrant can be starch, pregelatinized starch, low-substituted hydroxypropyl cellulose, microcrystalline cellulose, absolute lignocellulose, alginic acid, sodium carboxymethyl starch, croscarmellose sodium, guar gum, crosslinked polyvinylpyrrolidone, ion exchange resin, methylcellulose, carboxymethylcellulose sodium, and effervescent disintegrant composed of an organic acid (e.g., citric acid, and tartaric acid) and carbonate (e.g., sodium carbonate and sodium bicarbonate). The lubricant can be tartaric acid, magnesium stearate, calcium stearate, zinc stearate, talc, polyethylene glycol 4000, polyethylene glycol 6000, polyethylene glycol 8000, magnesium lauryl sulfate, sodium benzoate, sodium acetate, sodium chloride, sodium oleate, boric acid, leucine, adipic acid, fumaric acid, glycerol triacetate, polyoxyethylene monostearate, monolauryl saccharate, magnesium lauryl sulfate, and sodium lauryl sulfate. The glidant can be gas phase micro-powder silica gel, synthetic micro-powder silica gel, and magnesia. The surfactant can be sodium dodecylsulfate, poloxamer, Tweens, Spans, hexadecyl trimethylamine bromide, sodium lauryl sulfate, sodium stearate sulfonate, polyoxyethylene castor oil, and polyoxyethylene monostearate. The prescription can optionally contain flavor, including stevioside, fructose, sucrose, glucose, aspartame, protein sugar, xylitol, mannitol, sorbitol, lactose, maltitol, glycyrrhizin, sodium cyclohexylaminosulfonate, banana flavor, orange flavor, pineapple flavor, mint flavor, fennel, vanillin, lemon flavor, cherry flavor, and rose flavor. The prescription can also optionally contain wetting agent, i.e., aqueous or ethanol solution of different concentrations. The coating material useful in the pharmaceutical preparation of the present invention can be, for example, cellulose and derivatives thereof, acrylic resins, and polymers of ethylene.

The injection preparation includes injectable solution, concentrated solution for injection, or sterile powder for injection. The adjuvant is an additive meeting injection requirements, including pH adjusting agent, isotonic adjustment agent, anti-oxidant, chelating agent, and excipient in the sterile powder for injection. The pH adjusting agent includes hydrochloric acid, lactic acid, methanesulfonic acid, sodium hydroxide, sodium bicarbonate, phosphoric acid and salts thereof, acetic acid and salts thereof, citric acid and salts thereof, and amino acid and salts thereof; the isotonic adjustment agent includes glucose, sodium chloride, glycerol, and sodium sulfate; the excipient includes sorbitol, mannitol, dextran, lactose, sucrose, glucose, hydrolyzed gelatine, and sodium chloride; the anti-oxidant includes 0.01%-0.1% of sodium or potassium pyrosulfite, 0.01-0.5% of sodium sulfite, 0.01%-0.5% of sodium bisulphite, 0.01%-0.5% of sodium thiosulfate, 0.1-0.2% of sodium formaldehydesulfoxylate, 0.05%-0.1% of thiourea, 0.05%-0.2% of ascorbic acid, 0.1%-0.5% of thioglycerol, 0.01-0.2% of glutathione, 0.01%-0.5% of alanine, 0.01%-0.5% of cysteine, 0.01-0.1% of gallic acid and propyl or octyl ester thereof, 0.01-0.1% of tert-butyl p-hydroxyanisole, 0.01%-0.5% of di-tert-butyl p-cresol, 0.01%-0.1% of tocopherol α, β, and γ, 0.01%-0.5% of nordihydroguaiaretic acid, or 0.01%-0.5% of palmityl ascorbate; and the chelating agent includes disodium ethylene diamine tetraacetate, and calcium sodium ethylene diamine tetraacetate.

The compound of the present invention is useful in preparation of detoxification medicament for improving metabolism.

The present invention further provides a liquid chromatography for determining an optical purity of an optically active N-(α-mercaptopropionyl)glycine, including:

1) chromatographic conditions: the chromatography column has a stationary phase of a chiral column with 3,5-dimethylphenyl-carbamate glycopeptides and a mobile phase of n-hexane/ethanol/glacial acetic acid (80-95:5-20: 0.01-1.0), with (90:10:0.1) being preferred; the detection wavelength is 200 nm-230 nm, with 210 nm being preferred; and flow rate of the mobile phase is 0.2-3.0 ml/min, with 1.0 ml/min being preferred;

2) formulation of the sample solution: the sample R-(-)-N-(α-mercaptopropionyl)glycine or S-(+)-N-(α-mercaptopropionyl)glycine is formulated into a solution of 0.1-20 mg/ml (preferably 1 mg/ml) with an organic solvent; and the organic solvent is selected from n-propanol, i-propanol, ethanol, i-butanol, and methanol, with ethanol being preferred; and;

3) determination: the solution is injected into a high performance liquid chromatograph (HPLC), and the chromatogram is recorded and analyzed.

## BRIEF DESCRIPTION OF THE DRAWINGS

No drawings.

## DETAILED DESCRIPTION OF THE INVENTION

Embodiment 1: Preparation of R-(-)-N-(mercaptopropionyl)glycine

1) 54.3 g (0.5 mol) of R-(+)-2-chloropropionic acid, 60 g (0.504 mol) of thionyl chloride were added to a 100 ml dry reaction flask, and stirred at reflux for 4 h with moisture isolated. After the reaction was completed, excessive thionyl chloride was distilled out for reuse, and then the fraction having a bp of 95-105°C was collected, to get 55 g of R-(+)-2-chloropropionyl chloride as a colorless liquid with a yield of 86.6%.

2) 29.9 g (0.40 mol) of glycine, 21.2 g (0.20 mol) of anhydrous sodium carbonate, and 250 ml of water were added into a 1000 ml reaction flask, and stirred until dissolved. Cooling with an ice-salt bath, 50.6 g (0.40 mol) of R-(+)-2-chloropropionyl chloride was added dropwise with vigorously stirring, and a saturated solution of anhydrous sodium

carbonate was added at the same time, to make the reaction solution weak basic. After addition, stirring was continued for additional 3-5 h till the reaction was completed. The reaction solution was acidified to pH = 1 with concentrated hydrochloric acid, extracted with ethyl acetate, and then dried over anhydrous magnesium sulfate. The reaction solution was filtered and concentrated under reduced pressure till a crystal was precipitated. Then, the reaction solution was stood still, filtered, and dried, to get 38.6 g of R-(+)-2-chloropropionyl glycine as white small needle crystal. mp: 120-124°C, $[\alpha]_{n}^{20}$ = +23.8°C (water).

3) 26.5 g (0.11 mol) of sodium sulfide ($Na_2S\cdot 9H_2O$), 3.52 g (0.11 mol) of sublimed sulfur, and 120 ml of water were added to a 250 ml beaker, and heated with stirring until dissolved, to get a red brown solution of sodium disulfide for later use. 16.4 g (0.10 mol) of R-(+)-2-chloropropionyl glycine, and 5.6 g of anhydrous sodium carbonate were added into a 250 ml reaction flask, and then 100 ml of water was added slowly to prevent the generation of bubbles. After cooling to 0-10°C, the sodium disulfide solution was added dropwise, and then reacted for 10-15 h at 5-15°C. After the reaction was completed, the reaction solution was cooled to about 0°C, to which concentrated sulfuric acid was added dropwise, to adjust pH to approximately 1. Then, the reaction solution was filtered, and then 17 g of zinc powder was added in portion to the filtrate with stirring, and reacted for 3 h at normal temperature till the reaction was completed. The reaction solution was filtered, and then the filtrate was extracted with ethyl acetate, washed with saturated sodium chloride solution, and dried over anhydrous magnesium sulfate. The reaction solution was filtered, concentrated under reduced pressure, and stood still, to get a solid. The solid was filtered out and collected, and recrystallized with ethyl acetate, and then dried under vacuum, to get 8.4 g of R-(-)-N-(α-mercaptopropionyl) glycine as white crystalline solid. mp: 102-104°C, $[\alpha]_{n}^{20}$=-36.5°C (water), content: 99.3% (titrated with 0.1 mol/L of iodine titration solution ), related substance < 2% (thin layer chromatography: silica gel G thin plate, chloroform-acetone-glacial acetic acid (9:3: 1), developed with iodine vapor).

1HNMR (DMSO-D6) δ ppm: 1.35 (d, 3H); 2.79 (d, 1H); 3.54 (m, 1H); 3.77 (m, 2H); 8.25(t, 1H); 12.5(bs, 1H); MS (m/z) 163; element analysis $C_5H_9NO_3S$ (%): C 36.65, H 5.66, N 8.50, S 19.68.

Embodiment 2: Preparation of S-(+)-N-(α-mercaptopropionyl)glycine

1) 54.3 g (0.5 mol) of S-(-)-2-chloropropionic acid, 60 g (0.504 mol) of thionyl chloride were added to a 100 ml dry reaction flask, and stirred at reflux for 4 h with moisture isolated. After the reaction was completed, excessive thionyl chloride was distilled out for reuse, and then the fraction having a bp of 95-105°C was collected, to get 56 g of S-(-)-2-chloropropionyl chloride as a colorless liquid, with a yield of 88.2%.

2) 29.9 g (0.40 mol) of glycine, 21.2 g (0.20 mol) of anhydrous sodium carbonate, and 250 ml of water were added into a 1000 ml reaction flask, and stirred until dissolved. Cooling with an ice-salt bath, 50.6 g (0.40 mol) of S-(-)-2-chloropropionyl chloride was added dropwise with vigorously stirring, and a saturated solution of anhydrous sodium carbonate was added at the same time, to make the reaction solution weak basic. After addition, stirring was continued for additional 3-5 h till the reaction was completed. The reaction solution was acidified to pH = 1 with concentrated hydrochloric acid, extracted with ethyl acetate, and then dried over anhydrous magnesium sulfate. The reaction solution was filtered and concentrated under reduced pressure till a crystal was precipitated. Then, the reaction solution was stood still, filtered, and dried, to get 37.8 g of S-(-)-2-chloropropionyl glycine as white small needle crystal. mp: 120-124°C, $[\alpha]_{n}^{20}$=-24.2°C (water).

3) 26.5 g (0.11 mol) of sodium sulfide ($Na_2S\cdot 9H_2O$), 3.52 g (0.11 mol) of sublimed sulfur, and 120 ml of water were added to a 250 ml beaker, and heated with stirring until dissolved, to get a red brown solution of sodium disulfide for later use. 16.4 g (0.10 mol) of S-(-)-2-chloropropionyl glycine, and 5.6 g of anhydrous sodium carbonate were added into a 250 ml reaction flask, and then 100 ml of water was added slowly to prevent the generation of bubbles. After cooling to 0-10°C, the sodium disulfide solution was added dropwise, and then reacted for 10-15 h at 5-15°C. After the reaction was completed, the reaction solution was cooled to about 0°C, to which concentrated sulfuric acid was added dropwise, to adjust pH to approximately 1. The reaction solution was filtered, and then 17 g of zinc powder was added in portion to the filtrate with stirring, and reacted for 3 h at normal temperature till the reaction

was completed. Then, the reaction solution was filtered, and then the filtrate was extracted with ethyl acetate, washed with saturated sodium chloride solution, and dried over anhydrous magnesium sulfate. The reaction solution was filtered and concentrated under reduced pressure, and stood still, to get a solid. The solid was filtered and collected, and recrystallized with ethyl acetate, and then dried under vacuum, to get 8.2 g of S-(+)-N-(α-mercaptopropionyl) glycine as white crystalline solid. mp: 102-104°C, $[\alpha]_{n}^{20}$ =+37.5°C (water), content: 99.1% (titrated with 0.1 mol/L of iodine titration solution ), related substance < 2% (thin layer chromatography: silica gel G thin plate, chloroform-acetone-glacial acetic acid (9:3:1), developed with iodine vapor).

1HNMR (DMSO-D6) δ ppm: 1.40(d, 3H): 2.80 (d, 1H); 3.60 (m, 1H): 3.81 (m, 2H); 8.28 (t, 1H); 12.8 (bs, 1H); MS (m/z) 163: element analysis $C_5H_9NO_3S$ (%): C 36.68, H 5.65, N 8.53, S 19.65.

Embodiment 3: Preparation of S-(+)-N-(α-mercaptopropionyl)glycine

1) 54.3 g (0.5 mol) of S-(-)-2-chloropropionic acid, 60 g (0.504 mol) of thionyl chloride were added to a 100 ml dry reaction flask, and stirred at reflux for 4 h with moisture isolated. After the reaction was completed, excessive thionyl chloride was distilled out for reuse, and then the fraction having a bp of 95-105°C was collected to get 54.6 g of S-(-)-2-chloropropionyl chloride as a colorless liquid.

2) 29.9 g (0.40 mol) of glycine, 21.2 g (0.20 mol) of anhydrous sodium carbonate, and 250 ml of water were added into a 1000 ml reaction flask, and stirred until dissolved. Cooling with an ice-salt bath, 50.6 g (0.40 mol) of S-(-)-2-chloropropionyl chloride was added dropwise with vigorously stirring, and a saturated solution of anhydrous sodium carbonate was added at the same time, to make the reaction solution weak basic. After addition, stirring was continued for additional 3-5 h till the reaction was completed. The reaction solution was acidified to pH = 1 with concentrated hydrochloric acid, extracted with ethyl acetate, and then dried over anhydrous magnesium sulfate. The reaction solution was filtered and concentrated under reduced pressure till a crystal was precipitated. Then, the reaction solution was stood still, filtered, and dried, to get 38 g of S-(-)-2-chloropropionyl glycine as white small needle crystal.

3) 28.8 g (0.12 mol) of sodium sulfide ($Na_2S{\cdot}9H_2O$), 3.84 g (0.12 mol) of sublimed sulfur, and 120 ml of ethanol were added to a 250 ml beaker, and heated with stirring until dissolved, to get a red brown solution of sodium disulfide for later use. 18.3 g of (0.11 mol) of S-(-)-2-chloropropionyl glycine, and 6.3 g of anhydrous sodium carbonate were added into a 250 ml reaction flask, and then 100 ml of water was added slowly to prevent the generation of bubbles. After cooling to 0-10°C, the sodium disulfide solution was added dropwise, and then reacted for 12 h at 0-10°C. After the reaction was completed, the temperature was kept constant, and concentrated sulfuric acid was added dropwise, to adjust pH to approximately 1. The reaction solution was filtered, and then 17 g of zinc powder was added in portion to the filtrate with stirring, and reacted for 3 h at normal temperature till the reaction was completed. The reaction solution was filtered, and then the filtrate was extracted with ethyl acetate, washed with saturated sodium chloride solution, and dried over anhydrous magnesium sulfate. The reaction solution was filtered and concentrated under reduced pressure, and stood still to get a solid. The solid was filtered, collected, recrystallized with ethyl acetate, and then dried under vacuum, to get 8.1 g of S-(+)-N-(α-mercaptopropionyl)glycine as white crystalline solid. Content: 99.3% (titrated with 0.1 mol/L of iodine titration solution ), related substance < 2% (thin layer chromatography: silica gel G thin plate, chloroform-acetone-glacial acetic acid (9:3:1), developed with iodine vapor).

Embodiment 4: Determination of optical purity of R-(-)-N-(α-mercaptopropionyl)glycine

Instrument: HPLC, SPD-10Avp UV detector, LC-10AD pump
Mobile phase: n-hexane-ethanol-glacial acetic acid (90:10:0.1)
Flow rate: 1.0 ml/min
Chromatography column: CHIRALPAK AD-H (with 3,5-dimethylphenyl-carbamate glycopeptide as stationary phase)
Column temperature: 25°C
Detection wavelength: 210 nm
Sample concentration: 0.5 mg/ml
The optical activity of R-(-)-N-(α-mercaptopropionyl)glycine obtained in Embodiment 1 under the conditions above is

determined to be 99.3%.

Embodiment 5: Determination of optical purity of S-(+)-N-(α-mercaptopropionyl)glycine

Instrument: HPLC, SPD-10AvPUV detector, LC-10AD pump
Mobile phase: n-hexane-ethanol-glacial acetic acid (90:10:0.1)
Flow rate: 1.0ml/min
Chromatography column: CHIRALPAKAD-H (with 3,5-dimethylphenyl-carbamate glycopeptide as stationary phase)
Column temperature: 25°C
Detection wavelength: 210 nm
Sample concentration: 0.5 mg/ml
The optical activity of S-(+)-N-(α-mercaptopropionyl)glycine obtained in Embodiment 1 under the conditions above is determined to be 99.2%.

Embodiment 6: Preparation of R-(-)-N-(α-mercaptopropionyl)glycine arginine salt

10 g of R-(-)-N-(α-mercaptopropionyl)glycine obtained in Embodiment 1, 11.2 g of L-arginine, and 60 ml of 95% methanol were added into a reaction flask, stirred, heated to reflux, and reacted for 3 h. After the reaction was completed, the reaction solution was filtered while being hot, and then the filtrate was cooled to room temperature and placed in a freezer to precipitate a crystal by cooling. The crystal was filtered, collected, and dried to obtain 19.2 g of a white crystalline solid with a yield of 92.8%.

Embodiment 7: Preparation of S-(+)-N-(α-mercaptopropionyl)glycine lysine salt

10 g of S-(+)-N-(α-mercaptopropionyl)glycine obtained in Embodiment 2, 9.4 g of L-lysine, and 60 ml of 95% methanol were added into a reaction flask, stirred, heated to reflux, and reacted for 3 h. After the reaction was completed, the reaction solution was filtered while being hot, and then the filtrate was cooled to room temperature and placed in a freezer to precipitate a crystal by cooling. The crystal was filtered, collected, and dried to obtain 16.8 g of a white crystalline solid with a yield of 88.6%.

Embodiment 8: Preparation of R-(-)-N-(α-mercaptopropionyl)glycine sodium

1) 16.3 g (0.10 mol) of R-(-)-N(α-mercaptopropionyl)glycine, and 45 ml of methanol were placed in a reaction flask, to which 0.15 g of dithiothreitol was added at 15°C, and stirred for 15 min, and then 5 g of molecular sieve was added.

2) 4.65 g of NaOH was slowly added into the methanol solution above in 6 portions.

3) After addition, the reactants were reacted for 1 h at 15°C with stirring, and then for another 1 h at 20-25°C with stirring. After the reaction was completed, the molecular sieve was filtered off, and then the filtrate was added into 130 mL of acetone, and fully agitated to be homogeneously mixed.

4) The large amount of white precipitate present in the reaction solution was filtered by suction, and then the filter cake was dried at 80°C, to get 12.8 g of anhydrous R-(-)-N-(α-mercaptopropionyl)glycine sodium.

Embodiment 9: Preparation of S-(+)-N-(α-mercaptopropionyl)glycine sodium

1) 16.3 g (0.10 mol) of S-(+)-N-(α-mercaptopropionyl)glycine, and 45 ml of methanol were placed in a reaction flask, to which 0.15 g of dithiothreitol was added at 15°C, and stirred for 15 min, and then 5 g of molecular sieve was added.

2) 4.65 g of NaOH was slowly added into the methanol solution above in 6 portions.

3) After addition, the reactants were reacted for 1 h at 15°C with stirring, and then for another 1 h at 20-25°C with stirring. After the reaction was completed, the molecular sieve was filtered off, and then the filtrate was added into 130 mL of acetone, and fully agitated to be homogeneously mixed.

4) The large amount of white precipitate present in the reaction solution was filtered by suction, and then the filter cake was dried at 80°C, to get 13.1 g of anhydrous S-(+)-N-(α-mercaptopropionyl)glycine sodium.

Embodiment 10: Preparation of R-(-)-N-(α-mercaptopropionyl)glycine tablet

Formula Composition:

| | |
|---|---|
| R-(-)-N-(α-mercaptopropionyl)glycine | 100 g |
| Microcrystalline cellulose | 170 g |
| Pregelatinized starch | 60 g |
| 8% starch slurry | suitable amount |
| Sodium carboxymethyl starch | 7 g |
| Magnesium stearate | 3 g |

Preparation process: Taking the preparation of 1000 R-(-)-N-(α-mercaptopropionyl)glycine tablets as example, the raw and auxiliary materials were respectively pulverized and passed through a sieve of 100 mesh for later use; next, formula amounts of R-(-)-N-(α-mercaptopropionyl)glycine, microcrystalline cellulose, and pregelatinized starch were weighted, and homogeneously mixed to prepare a soft material with 8% starch slurry, which was granulated with a sieve of 20 mesh, dried, and then sized with a sieve of 18 mesh; afterwards, formula amounts of sodium carboxymethyl starch and magnesium stearate were added, homogeneously mixed, and then pressed into tablets. The weight of each tablet is about 345 mg.

Embodiment 11: Preparation of film-coated R-(-)-N-(α-mercaptopropionyl)glycine tablet

The naked tablet obtained in Embodiment 10 was coated with a formulated 8% gastric soluble OPADRY (OY-C-7000A) solution in ethanol in a high efficient coating pan; and the amount of the coating powder is 2.0-3.0% by weight of the naked tablet.

Embodiment 12: Preparation of enteric soluble R-(-)-N-(α-mercaptopropionyl)glycine tablet

The naked tablet obtained in Embodiment 10 was coated with a formulated 8% enteric soluble OPADRY solution in ethanol in a high efficient coating pan; and the amount of the coating powder is 4.0-5.0% by weight of the naked tablet.

Embodiment 13: Preparation of slow release R-(-)-N-(α-mercaptopropionyl)glycine tablet

Formula Composition:

| | |
|---|---|
| R-(-)-N-(α-mercaptopropionyl)glycine | 150 g |
| Hydroxypropylmethyl cellulose (K15M) | 150 g |
| Lactose | 50 g |
| 3% povidone solution in 80% ethanol | suitable amount |
| Magnesium stearate | 5 g |

Preparation process: Taking the preparation of 1000 slow release R-(-)-N-(α-mercaptopropionyl)glycine tablets as example, the raw and auxiliary materials were respectively pulverized and passed through a sieve of 100 mesh for later use; next, formula amounts of R-(-)-N-(α-mercaptopropionyl)glycine, hydroxypropylmethyl cellulose, and lactose were weighted, and homogeneously mixed to prepare a soft material with 3% povidone solution in 80% ethanol, which was granulated with a sieve of 20 mesh, dried, and then sized with a sieve of 18 mesh; afterwards, formula amounts of magnesium stearate were added, homogeneously mixed, and then pressed into tablets. The weight of each tablet is about 360 mg.

Embodiment 14: Preparation of R-(-)-N-(α-mercaptopropionyl)glycine dispersible tablet

Formula Composition:

| | |
|---|---|
| R-(-)-N-(α-mercaptopropionyl)glycine | 100 g |
| Crosslinked polyvinylpyrrolidone | 15 g |
| Microcrystalline cellulose | 180 g |
| Mannitol | 50 g |
| 60% ethanol solution | suitable amount |
| Sodium dodecyl sulfate | 0.2 g |
| Micro-powder silica gel | 5 g |
| Magnesium stearate | 3 g |
| Stevioside | 5 g |

Preparation process: Taking the preparation of 1000 R-(-)-N-(α-mercaptopropionyl)glycine dispersible tablets as example, the raw and auxiliary materials were respectively pulverized and passed through a sieve of 100 mesh for later use; next, formula amounts of R-(-)-N-(α-mercaptopropionyl)glycine, microcrystalline cellulose, and mannitol were weighted, and homogeneously mixed to prepare a soft material with 60% ethanol solution, which was granulated, dried, and then sized; afterwards, formula amounts of crosslinked polyvinylpyrrolidone, sodium dodecyl sulfate, micro-powder silica gel, magnesium stearate, and stevioside were added, homogeneously mixed, and then pressed into tablets. The weight of each tablet is about 360 mg.

Embodiment 15: Preparation of R-(-)-(α-mercaptopropionyl)glycine capsule

Formula Composition:

| | |
|---|---|
| R-(-)-N-(α-mercaptopropionyl)glycine | 150 g |
| Starch | 45 g |
| 5% povidone solution in 60% ethanol | suitable amount |
| Magnesium stearate | 2 g |

Preparation process: Taking the preparation of 1000 R-(-)-N-(α-mercaptopropionyl)glycine capsules as example, the raw and auxiliary materials were respectively pulverized and passed through a sieve of 100 mesh for later use; next, formula amounts of R-(-)-N-(α-mercaptopropionyl)glycine, and starch were weighted, and homogeneously mixed to prepare a soft material with 5% povidone solution in 60% ethanol, which was granulated, dried, and then sized; afterwards, formula amounts of magnesium stearate was added, homogeneously mixed, and then filled into a capsule.

Embodiment 16: Preparation of R-(-)-N-(α-mercaptopropionyl)glycine granule

Formula Composition:

| | |
|---|---|
| R-(-)-N-(α-mercaptopropionyl)glycine | 100 g |
| Mannitol | 350 g |
| Sucrose | 350 g |
| Sodium carboxymethyl cellulose | 50 g |
| Aspartame | 10 g |
| Maltdextrin | 140 g |
| 5% povidone solution in 80% ethanol | suitable amount |
| Flavor | 1 g |

Preparation process: Taking the preparation of 1000 packages of R-(-)-N-(α-mercaptopropionyl)glycine granule as example, the raw and auxiliary materials were respectively pulverized and passed through a sieve of 100 mesh for later use; next, the flavor was added into 5% povidone solution in 80% ethanol, and then formula amounts of R-(-)-N-(α-mercaptopropionyl)glycine, sucrose, mannitol, sodium carboxymethyl cellulose, aspartame, and maltdextrin were weighted to prepare a soft material with 5% povidone solution in 80% ethanol, which was granulated with a sieve of 16 mesh, dried, and then sized with a sieve of 14 mesh; afterwards, the granulates were sieved to remove the fine powder with a sieve of 60 mesh, and packaged. The weight of each package is about 1 g.

Embodiment 17: Preparation of injectable R-(-)-N-(α-mercaptopropionyl)glycine solution

Formula Composition:

| | |
|---|---|
| R-(-)-N-(α-mercaptopropionyl)glycine | 100 g |
| Water for injection | made up to 2000 ml |

Preparation process: Taking the preparation of 1000 ampoules of injectable R-(-)-N-(α-mercaptopropionyl) glycine solution as example, formula amount of R-(-)-N-(α-mercaptopropionyl)glycine was added into 1600 ml of water for injection, and stirred until completely dissolved; then additional water for injection was added to make up to 2000 ml, and uniformly stirred; afterwards, 0.1 % of activated carbon for refinement of injection was added, and uniformly stirred; the drug solution was processed with titanium rod to remove active carbon, and then finely filtered with 0.45 μm and 0.22 μm micro membranes in sequence to a satisfactory clarification; then the solution was detected for intermediates, filled into 2 ml ampoules at 2 ml specification under nitrogen flow if eligible, and then sterilized.

Embodiment 18: Preparation of R-(-)-N-(α-mercaptopropionyl)glycine injection

Formula Composition:

| | |
|---|---|
| R-(-)-N-(α-mercaptopropionyl)glycine | 100 g |
| Dextran 40 | 50 g |
| Sodium bisulphate | 1 g |
| Disodium ethylenediamine tetraacetate | 0.2 g |

Preparation process: Taking the preparation of 1000 vials of R-(-)-N-(α-mercaptopropionyl)glycine for injection as example, formula amounts of R-(-)-N-(α-mercaptopropionyl)glycine, dextran 40, sodium bisulphate, and disodium ethylenediamine tetraacetate were added into 1600 ml of water for injection, and stirred until completely dissolved; next, the pH of the solution was adjusted to 1.5-2.5 with 1mol/L hydrochloric acid, and then additional water for injection was added to make up to 2000 ml, and uniformly stirred; afterwards, 0.1% of activated carbon for refinement of injection was added, and uniformly stirred; the drug solution was processed with titanium rod to remove active carbon, and then finely filtered with 0.45 μm and 0.22 μm micro membranes in sequence and sterilized under aseptic conditions; the drug solution was detected for the presence of intermediates, pH, content, and clarification, and filled into a penicillin bottle at 2 ml specification if eligible, partially stoppered, freeze dried, stoppered and capped, and then light inspected.

Embodiment 19: Preparation of S-(+)-N-(α-mercaptopropionyl)glycine tablet

Formula Composition:

| | |
|---|---|
| S-(+)-N-(α-mercaptopropionyl)glycine | 100 g |
| Microcrystalline cellulose | 170 g |
| Pregelatinized starch | 60 g |
| 8% starch slurry | suitable amount |

(continued)

| | |
|---|---|
| Sodium carboxymethyl starch | 7 g |
| Magnesium stearate | 3 g |

Preparation process: Taking the preparation of 1000 S-(+)-N-(α-mercaptopropionyl)glycine tablets as example, the raw and auxiliary materials were respectively pulverized and passed through a sieve of 100 mesh for later use; next, formula amounts of S-(+)-N-(α-mercaptopropionyl)glycine, microcrystalline cellulose, and pregelatinized starch were weighted, and homogeneously mixed to prepare a soft material with 8% starch slurry, which was granulated with a sieve of 20 mesh, dried, and then sized with a sieve of 18 mesh; afterwards, formula amounts of sodium carboxymethyl starch and magnesium stearate were added, homogeneously mixed, and then pressed into tablets. The weight of each tablet is about 345 mg.

Embodiment 20: Preparation of film-coated S-(+)-N-(α-mercaptopropionyl)glycine tablet

The naked tablet obtained in Embodiment 19 was coated with a formulated 8% gastric soluble OPADRY (OY-C-7000A) solution in ethanol in a high efficient coating pan; and the amount of the coating powder used is 2.0-3.0% by weight of the naked tablet.

Embodiment 21: Preparation of enteric soluble S-(+)-N-(α-mercaptopropionyl)glycine tablet

The naked tablet obtained in Embodiment 19 was coated with a formulated 8% enteric soluble OPADRY solution in ethanol in a high efficient coating pan; and the amount of the coating powder used is 4.0-5.0% by weight of the naked tablet.

Embodiment 22: Preparation of slow release S-(+)-N-(α-mercaptopropionyl)glycine tablet

Formula Composition:

| | |
|---|---|
| S-(+)-N-(α-mercaptopropionyl)glycine | 150 g |
| Hydroxypropylmethyl cellulose (K15M) | 150 g |
| Lactose | 50g |
| 3% povidone solution in 80% ethanol | suitable amount |
| Magnesium stearate | 5 g |

Preparation process: Taking the preparation of 1000 slow release S-(+)-N-(α-mercaptopropionyl)glycine tablets as example, the raw and auxiliary materials were respectively pulverized and passed through a sieve of 100 mesh for later use; next, formula amounts of S-(+)-N-(α-mercaptopropionyl)glycine, hydroxypropylmethyl cellulose, and lactose were weighted, and homogeneously mixed to prepare a soft material with 3% povidone solution in 80% ethanol, which was granulated with a sieve of 20 mesh, dried, and then sized with a sieve of 18 mesh; afterwards, formula amount of magnesium stearate was added, homogeneously mixed, and then pressed into tablets. The weight of each tablet is about 360 mg.

Embodiment 23: Preparation of S-(+)-N-(α-mercaptopropionyl)glycine dispersible tablet

Formula Composition:

| | |
|---|---|
| S-(+)-N-(α-mercaptopropionyl)glycine | 100 g |
| Crosslinked polyvinylpyrrolidone | 15 g |
| Microcrystalline cellulose | 80 g |
| Mannitol | 50 g |
| 60% ethanol solution | suitable amount |

(continued)

| | |
|---|---|
| Sodium dodecyl sulfate | 0.2 g |
| Micro-powder silica gel | 5 g |
| Magnesium stearate | 3 g |
| Stevioside | 5 g |

Preparation process: Taking the preparation of 1000 R-(-)-N-(α-mercaptopropionyl)glycine dispersible tablet as example, the raw and auxiliary materials were respectively pulverized and passed through a sieve of 100 mesh for later use; next, formula amounts of S-(+)-N-(mercaptopropionyl)glycine, microcrystalline cellulose, and mannitol were weighted, and homogeneously mixed to prepare a soft material with 60% ethanol solution, which was granulated, dried, and then sized; afterwards, formula amounts of crosslinked polyvinylpyrrolidone, sodium dodecyl sulfate, micro-powder silica gel, magnesium stearate, and stevioside were added, homogeneously mixed, and then pressed into tablets. The weight of each tablet is about 360 mg.

Embodiment 24: Preparation of S-(+)-N-(α-mercaptopropionyl)glycine capsule

Formula Composition:

| | |
|---|---|
| S-(+)-N-(α-mercaptopropionyl)glycine | 150 g |
| Starch | 45 g |
| 5% povidone solution in 60% ethanol | suitable amount |
| Magnesium stearate | 2 g |

Preparation process: Taking the preparation of 1000 S-(+)-N-(α-mercaptopropionyl)glycine capsules as example, the raw and auxiliary materials were respectively pulverized and passed through a sieve of 100 mesh for later use; next, formula amounts of S-(+)-N-(α-mercaptopropionyl)glycine, and starch were weighted, and homogeneously mixed to prepare a soft material with 5% povidone solution in 60% ethanol, which was granulated, dried, and then sized; afterwards, formula amounts of magnesium stearate was added, homogeneously mixed, and then filled into a capsule.

Embodiment 25: Preparation of S-(+)-N-(α-mercaptopropionyl)glycine granule

Formula Composition:

| | |
|---|---|
| S-(+)-N-(α-mercaptopropionyl)glycine | 100 g |
| Mannitol | 350 g |
| Sucrose | 350 g |
| Carboxymethylcellulose sodium | 50 g |
| Aspartame | 10 g |
| Maltdextrin | 140 g |
| 5% povidone solution in 80% ethanol | suitable amount |
| Flavor | 1 g |

Preparation process: Taking the preparation of 1000 packages of S-(+)-N-(α-mercaptopropionyl)glycine granule as example, the raw and auxiliary materials were respectively pulverized and passed through a sieve of 100 mesh for later use; next, the flavor was added into 5% povidone solution in 80% ethanol, and then, formula amounts of S-(+)-N-(α-mercaptopropionyl)glycine, mannitol ,sucrose, sodium carboxymethyl cellulose, aspartame, and maltdextrin were weighted to prepare a soft material with 5% povidone solution in 80% ethanol, which was granulated with a sieve of 16 mesh, dried, and then sized with a sieve of 14 mesh; afterwards, it was sieved to remove the fine powder with a sieve of 60 mesh, and packaged. The weight of each package is about 1 g.

Embodiment 26: Preparation of injectable S-(+)-N-(α-mercaptopropionyl)glycine solution

Formula Composition:

| | |
|---|---|
| S-(+)-N-(α-mercaptopropionyl)glycine | 100 g |
| Sodium pyrosulfite | 1 g |
| Disodium ethylene diamine tetraacetate | 0.2 g |
| Water for injection | made up to 2000 ml |

Preparation process: Taking the preparation of 1000 ampoules of injectable S-(+)-N-(α-mercaptopropionyl) glycine solution as example, formula amounts of S-(+)-N-(α-mercaptopropionyl)glycine, sodium pyrosulfite and disodium ethylenediamine tetraacetate were added into 1600 ml of water for injection, and stirred until completely dissolved; then additional water for injection was added to make up to 2000 ml, and uniformly stirred; afterwards, 0.1% of activated carbon for refinement of injection was added, and uniformly stirred; the drug solution was processed with titanium rod to remove active carbon, and then finely filtered with 0.45 μm and 0.22 μm micro membranes in sequence to a satisfactory clarification; then the solution was detected for intermediates, filled into 2 ml ampoules at 2 ml specification under nitrogen flow if eligible, and then sterilized.

Embodiment 27: Preparation of S-(+)-N-(α-mercaptopropionyl)glycine for injection

Formula Composition:

| | |
|---|---|
| S-(+)-N-(α-mercaptopropionyl)glycine | 100 g |
| Dextran 40 | 50 g |

Preparation process: Taking the preparation of 1000 vials of S-(+)-N-(α-mercaptopropionyl)glycine for injection as example, formula amounts of S-(+)-N-(α-mercaptopropionyl)glycine, and dextran 40 were added into 1600 ml of water for injection, and stirred until completely dissolved; next, the pH of the solution was adjusted to 1.5-2.5 with l mol/L hydrochloric acid or 1 mol/L sodium hydroxide solution, and then additional water for injection was added to make up to 2000 ml, and uniformly stirred; afterwards, 0.1 % of activated carbon for refinement of injection was added, and uniformly stirred; the drug solution was processed with titanium rod to remove active carbon, and then finely filtered with 0.45 μm and 0.22 μm micro membranes in sequence and sterilized under aseptic conditions; the solution was detected for the presence of intermediates, pH, content, and clarification, and filled into a penicillin at 2 ml specification if eligible, partially stoppered, freeze dried, stoppered and capped, and then light inspected.

Embodiment 28: Effect of optically active N-(α-mercaptopropionyl)glycine administrated by intravenous injection on serum biochemical indicators and liver indexes of mice with liver injury induced by acetamidophenol

The animals were randomly divided into the following 5 groups of 10 animals according to weight: solvent control group, model group (acetamidophenol, 400 mg/kg, 10 ml/kg, intraperitoneal injection), tiopronin group (MPG), R-(-)-N-(α-mercaptopropionyl)glycine group, and S-(+)-N-(α-mercaptopropionyl)glycine group. After fasting for 12 h, except the solvent control group and model group were given saline by intravenous injection, the other test groups were intravenously injected drugs, and then intraperitoneally injected 400/mg/kg of acetamidophenol immediately, to induce acute liver cell injury. The animals were fed with food at 6 hours after modeling, and then fasted for 16 hours; and at 24 hours after modeling, blood were sampled by eye ball removal, centrifuged, and collected for serum. ALT, and AST were determined following the method of the kit. 10% of liver homogenate was prepared, to measure GSH. The liver and spleen were taken at the same time, weighted, and calculated for organcoefficient. The results are shown in Tables 1 and 2.

Table 1. Effect of MPG and optical isomer thereof administrated by intravenous injection on serum biochemical indicators of mice with liver injury induced by acetamidophenol

| Group | Dosage (mg/kg) | Number of animals | Liver coefficient (mg/g) | ALT (Karl-Fischer unit) | AST (Karl-Fischer unit) | Serum total protein (mg/ml) |
|---|---|---|---|---|---|---|
| Solvent control group | - | 12 | 5. 405±0.508 | 30.5±18.1 | 30.0±6.5 | 47.5±4.7 |
| Model group | - | 10 | 6.139±0.683 | 5761.8±2761.8 | 1682.8±112.0 | 42.9±8.1 |
| Tiopronin group | 800 | 8 | 5.804±0.598 | 648.1±412.1 | Not detected due to insufficient serum | Not detected due to insufficien t serum |
| S-(+)-N-(α-mercaptopropionyl) glycin | 800 | 12 | 6.201±0.56 | 2880.2±2578. | 1201.2±1123. | 43.7±5.7 |
| e group | | | 7 | 5 | 0 | |
| R-(-)-N-(α-mercaptopropionyl) glycine group | 800 | 12 | 5.816±0.61 3 | 1812.1±1780. 1 | 591.3±528.0 | 43.8±7.4 |
| *P<005, **P<0.01 compared with the model group | | | | | | |

It can be known from Table 1 that, compared with the solvent control group, the liver coefficient of the mice in the model group is significantly increased, and inhibition of the elevation of serum ALT is apparent in all the dosed groups, in which the function in the R-(-)-N-(α-mercaptopropionyl)glycine group is higher than that in the S-(+)-N-(α-mercaptopropionyl)glycine group. The ALT value of tiopronin group is lower than that of R-(-)-N-(α-mercaptopropionyl) glycine group, but in the experiment, after modeling, 4 mouse dead in the tiopronin group die, 2 mouse dead in the model group, and no death occurs in the R-(-)-N-(α-mercaptopropionyl)glycine group. Therefore, the protection against liver injury in mice induced by acetamidophenol in R-(-)-N-(α-mercaptopropionyl)glycine group is superior to that in tiopronin group.

Table 2. Effect of MPG and optical isomer thereof administrated by intravenous injection on liver indexes of mice with liver injury induced by acetamidophenol

| Group | Dosage (mg/ kg ) | Number of animal | Liver total protein (mg/ml) | Liver albumin (mg/ml) | GSH (mol/L) | MDA (nmol/g wet weight) |
|---|---|---|---|---|---|---|
| Solvent control group | - | 12 | 17.4±3.6 | 10.0±1.9 | 1.20±0.15 | 288.9±84.5 |
| Model group | - | 10 | 13.0±1.5 | 8.3±0.7 | 0.93±0.12 | 452.2±132. 8 |
| Tiopronin group | 800 | 8 | 15.7±1.4 | 9. 6±1.1 | 0.99±0.16 | 362.4±98.6 |
| S-(+)-N-(α-mercaptopropionyl) glycine group | 800 | 12 | 16. 2±2.9 | 9. 1±2.0 | 0.99±0.16 | 440.0±134.6 |
| R-(-)-N-(α-mercaptopropionyl) glycine group | 800 | 12 | 16. 5±1. 7 | 9.2+1.3 | 1.11±0.16 | 321.7±72.9 |
| *P<0.05, **P<0.01 compared with the model group | | | | | | |

It can be known from Table 2 that, compared with the model group, the content of liver total protein is significantly increased in all the dosed groups, liver GSH content is obviously increased and MDA content is decreased in the R-(-)-N-(α-mercaptopropionyl)glycine group, but no such effects are present in the S-(+)-N-(α-mercaptopropionyl)glycine group and tiopronin group. The results indicate that R-(-)-N-(α-mercaptopropionyl)glycine can provide better protection against liver injury.

Embodiment 29: Protection of N-(α-mercaptopropionyl)glycine and optical isomer thereof against liver injury in rats induced by carbon tetrachloride

The animals were randomly divided into the following 5 groups of 10 animals according to weight: solvent control group, model group (50% $CCl_4$, 2 ml/kg, intraperitoneal injection), tiopronin group (MPG), R-(-)-N-(α-mercaptopropionyl)glycine group, and S-(+)-N-(α-mercaptopropionyl)glycine group. Except the solvent control group was given olive oil by intraperitoneal injection, the other test groups were intragastrically administrated drugs for 4 days, once daily, intraperitoneally injected $CCl_4$ once on the fifth day to induce acute liver cell injury, treated with drugs 30 min before modeling (except for the model control group), and then administrated drugs once 2 hours after modeling. Then, the animals were fasted for 16 h, blood was sampled from retrobulbar venous plexus 24 hours after injecting $CCl_4$, centrifuged, and collected for serum, and ALT, AST, albumin were determined following the method of the kit. The results are shown in Tables 3, and 4. The left lobe of liver was taken to prepare 10% of liver homogenate and measure GSH and MDA contents. The results are shown in Table 5.

Table 3. Effects of MPG and optical isomer thereof on ALT, and AST of rats with liver injury induced by $CCl_4$

| Group | Dosage (mg/kg, ig) | ALT (Karl-Fischer unit) | AST(Karl-Fischer unit) |
|---|---|---|---|
| Solvent control group Model group | - | 327.6±11.4 | 117.6±46.5 |
| | - | 1020.1±275.0 | 1448.1±362.5 |
| Tiopronin group | 450 | 748.6±260.2 | 1126.0±159.2 |
| S-(+)-N-(α-mercaptopropionyl)glycine group | 450 | 532.8±352.7 | 818.3±347.0 |
| R-(-)-N-(α-mercaptopropionyl)glycine group | 450 | 432.8±252.6 | 718.7±336.2 |
| *P<0.05, **P<0.01 compared with the model group | | | |

It can be known from Table 3 that, all the three drugs can significantly decrease the ALT and AST level, in which the decrease in the S-(+)-N-(α-mercaptopropionyl)glycine group and the R-(-)-N-(α-mercaptopropionyl)glycine group is higher than that in the tiopronin group.

Table 4. Effects of MPG and optical isomer thereof on serum protein content of rats with liver injury induced by $CCl_4$

| Group | Dosage (mg/kg, ig) | Total protein (mg/ml) | Albumin (mg/ml) | Globulin (mg/ml) | A/G |
|---|---|---|---|---|---|
| Solvent control group | - | 76.2±5.14 | 30.1±2.23 | 46.1±5.13 | 0.660±0.087 |
| Model group | - | 59.1±7.56 | 26.8±2.74 | 32.3±4.99 | 0.837±0.059 |
| Tiopronin group | 450 | 59.8±6.26 | 26.8±1.98 | 32.9±4.79 | 0.825±0.101 |
| S-(+)-N-(α-mercaptopropionyl) glycine group | 450 | 64.7±6.40 | 27.5±1.46 | 37.2-±5.06 | 0.747±0.073 |
| R-(-)-N-(α-mercaptopropionyl) glycine group | 450 | 70.6±3.63 | 30.3±1.20 | 40.3±2.98 | 0.754±0.050 |
| **P<0.01 compared with the model group | | | | | |

It can be known from Table 4 that, compared with the model group, in the R-(-)-N-(α-mercaptopropionyl)glycine group, the serum total protein, albumin and globulin contents are obviously increased, and A/G value is decreased; A/G value is also decreased in the S-(+)-N-(α-mercaptopropionyl)glycine group; but no obvious effect on the indexes are detected in the tiopronin group, which corresponds to the results obtained in the model group.

## Claims

1. An optically active N-(α-mercaptopropionyl)glycine, being levo or dextro N-(α-mercaptopropionyl)glycine;

wherein:

the levo N-(α-mercaptopropionyl)glycine is R-(-)-N-(α-mercaptopropionyl)glycine:

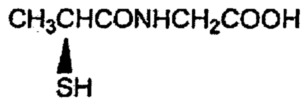

of R-configuration, or a pharmaceutically acceptable salt or ester thereof; and
the dextro N-(α-mercaptopropionyl)glycine is S-(+)-N-(α-mercaptopropionyl)glycine:

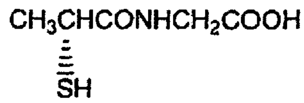

of S-configuration, or a pharmaceutically acceptable salt or ester thereof.

**2.** The optically active N-(α-mercaptopropionyl)glycine according to claim 1, wherein the salt is an amino acid salt or a metal salt, the amino acid salt has a general structural formula below:

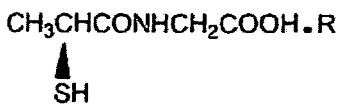
or
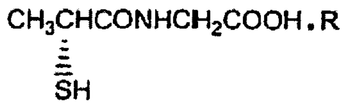
,

wherein R is an amino acid selected from arginine, lysine, glycine, aspartic acid, alanine, phenylalanine, leucine, isoleucine, ornithine, cystine, cysteine, tyrosine, valine, serine, histidine, threonine, tryptoophan, methionine, methionine, proline, glutamic acid, or hydroxyproline; and
the metal salt has a general structural formula below:

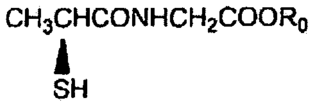
or
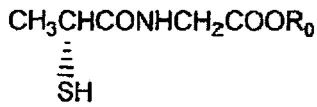
,

wherein $R_0$ is potassium, or sodium.

**3.** The optically active N-(α-mercaptopropionyl)glycine according to claim 1, wherein the ester has a general structural formula below:

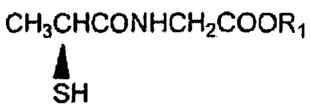
or
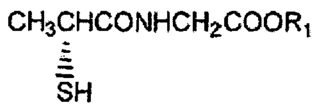
,

wherein $R_1$ is a linear $C_1$-$C_5$ alkyl group.

**4.** A method for preparing the optically active N-(α-mercaptopropionyl)glycine according to claim 1, comprising:

reacting R-(+)-2-chloropropionic acid with thionyl chloride to get R-(+)-2-chloropropionyl chloride; next, reacting R-(+)-2-chloropropionyl chloride with glycine under a weak basic condition to get R-(+)-2-chloropropionyl glycine; and then, reacting R-(+)-2-chloropropionyl glycine with sodium disulfide to get R-(-)-N-(α-mercaptopropionyl)glycine; or comprising:

reacting S-(-)-2-chloropropionic acid with thionyl chloride to get S-(-)-2-chloropropionyl chloride; next, reacting S-(-)-2-chloropropionyl chloride with glycine under a weak basic condition to get S-(-)-2-chloropropionyl glycine; and then, reacting S-(-)-2-chloropropionyl glycine with sodium disulfide to get S-(+)-N-(α-mercaptopropionyl) glycine;
wherein the sodium disulfide is prepared by reacting sodium sulfide with sublimed sulfur.

**5.** A use of the optically active N-(α-mercaptopropionyl)glycine according to claim 1, or a pharmaceutically acceptable salt or ester thereof in preparing a medicament for treating acute and chronic liver diseases, peripheral blood leukopenia caused by chemoradiation therapy, early senile cataract, or vitreous opacities, wherein the acute and chronic liver diseases include acute and chronic hepatitis, viral hepatitis, alcoholic hepatitis or drug induced hepatitis, heavy metal toxic hepatitis, fatty liver, acute and chronic liver injury, and cirrhosis; and the medicament is an orally administrated preparation or an injection preparation.

**6.** The use in preparing a medicament according to claim 5, wherein the medicament comprising R-(-)-N-(α-mercaptopropionyl)glycine or S-(+)-N-(α-mercaptopropionyl)glycine, or a pharmaceutically acceptable salt or ester thereof as active ingredient, and a pharmaceutically acceptable adjuvant.

**7.** The use in preparing a medicament according to claim 5, wherein the preparation is an orally administrated preparation, comprising a general oral preparation, a rapid release oral preparation, or a slow release preparation, and the general oral preparation comprises tablet, capsule, granule, chewable tablet, or effervescent tablet, the rapid release oral preparation is dispersible tablet, or orally disintegrating tablet, and the slow release preparation is slow release tablet or slow release pellet.

**8.** The use in preparing a medicament according to claim 5, wherein the preparation is an injection preparation, comprising injectable solution, concentrated solution for injection, or sterile powder for injection.

**9.** The use in preparing a medicament according to claim 6 or 7, wherein the adjuvant in the oral pharmaceutical preparation comprises a filler, a binder, or a disintegrant, wherein the weight contents of the filler and the disintegrant are 10-60%, and 2-30% respectively; and a glidant, a lubricant, and/or a surfactant optionally, wehrein the weight contents of the gilidant, the lubricant, and the surfactant are 0.1-5%, 0.1-5%, and 0.005-1% respectively.

**10.** The use in preparing a medicament according to claim 6 or 8, wherein the adjuvant in the injection preparation is an additive meeting injection requirements, comprising a pH adjusting agent, an isotonic adjustment agent, an antioxidant, a chelating agent, and/or an excipient.

**11.** A liquid chromatography for determining an optical purity of an optically active N-(α-mercaptopropionyl)glycine, comprising:

(1) chromatographic conditions: a chromatography column is a chiral column with 3,5-dimethylphenyl-carbamate glycopeptide as stationary phase and n-hexane/ethanol/glacial acetic acid (80-95:5-20:0.01-1.0), with (90:10: 0.1) being preferred as mobile phase; the detection wavelength is 200 nm-230 nm, with 210 nm being preferred; flow rate of mobile phase is 0.2-3.0 ml/min, with 1.0 ml/min being preferred;
(2) formulation of sample solution: sample R-(-)-N-(α-mercaptopropionyl)glycine or S-(+)-N-(α-mercaptopropionyl)glycine is formulated in an organic solvent to give a solution of 0.1-20 mg/ml (preferably 1 mg/ml); wherein the organic solvent is selected from n-propanol, i-propanol, ethanol, i-butanol, and methanol, with ethanol being preferred; and
(3) determination: the solution is injected into a high performance liquid chromatograph (HPLC), the chromatogram is recorded and analyzed.

**12.** A method for determining a pharmacokinetic profile of an optically active N-(α-mercaptopropionyl)glycine, comprising determining the plasma drug concentration in an test animal with high performance liquid chromatography-mass spectrometry (HPLC-MS), wherein:

a process for treat a plasma sample to formulate an injected solution comprises plasma acidification, extraction, and then derivatization, and is as follows:

1. plasma acidification, wherein the acid used for acidification comprises hydrochloric acid, phosphoric acid, perchloric acid, or acetic acid, with hydrochloric acid being preferred, and hydrochloric acid of 1 mol/L being more preferred; and wherein the volume ratio of the acid to the plasma sample is (150 μl-250 μl):(2 ml-4 ml), with 200 μl:3 ml being preferred;
2. extraction of the acidified plasma, wherein the organic solvent used for extraction comprises ethyl acetate, chloroform, trichloromethane, diethyl ether, or n-hexane, with ethyl acetate being preferred; and
3. derivatization of the extract, wherein the useful derivatization reagent compirses phenyl isothiocyanate and 2,3,4,6-tetra-O-acetyl-β-D-pyranoglucose isothiocyanate (GITC) solution, with GITC solution being preferred, GITC solution of 2 mg/ml being more preferred, and solution of 2 mg/ml GITC in tetrahydrofuran being most preferred; wherein the derivatization temperature is 15-45°C, with 25-35°C being preferred, and 30°C being more preferred; and where the derivatization time is 10-30 min, with 15-25 min being preferred, and 20 min being more preferred;

chromatographic conditions comprise:

mobile phase A: methanol; mobile phase B: an aqueous solution, containing 0.05-0.20 mmol/L of sodium chloride and 5.0-6.0 mmol/L of formic acid, with 0.10 mmol/L of sodium chloride and 5.3 mmol/L of formic acid being preferred, wherein the ratio of A to B is (40-50:50-60, with 44:56 being preferred).
mass spectrometric conditions comprise:

ionization mode: electrospray ionization; selective ion detection; curved desolvation line (CDL); temperature: 200°C-300°C, with 250°C being preferred; heating block temperature: 150°C-250°C, with 200°C being preferred; CDL voltage: 20V-30V. with 25V being preferred; detection voltage: +1.2 kV-+1.8 kV, with +1.50 kV being preferred; flow rate of atomizing gas: 1.2 L/min-1.8 L/min, with 1.5 L/min being preferred; flow rate of drying gas: 1.5 L/min-2.5 L/min, with 2.0 L/min being preferred; detected ion: derivative of test drug [M+Na]+(m/z): 575.20; internal standard: derivative of N-isobutanoyl-D-cysteine (NIDC) [M+Na]+(m/z): 603.05; as long as the separation degree of the internal standard peak from the major peak meets the requirements of Chinese Pharmacopoeia.

## INTERNATIONAL SEARCH REPORT

International application No.
PCT/CN2007/003859

A. CLASSIFICATION OF SUBJECT MATTER

See extra sheet

According to International Patent Classification (IPC) or to both national classification and IPC

B. FIELDS SEARCHED

Minimum documentation searched (classification system followed by classification symbols)

IPC C07C323/-,C07C319/-,A61K31/-,A61P39/-,A61P43/-

Documentation searched other than minimum documentation to the extent that such documents are included in the fields searched

Electronic data base consulted during the international search (name of data base and, where practicable, search terms used)

CNKI,CPRS,WPI,PAJ,EPODOC,STN,CA,thiopronin?,glycin?

C. DOCUMENTS CONSIDERED TO BE RELEVANT

| Category* | Citation of document, with indication, where appropriate, of the relevant passages | Relevant to claim No. |
|---|---|---|
| X | JP2204478 A（SANTEN PHARM CO LTD) ,14 Aug.1990（14.08.1990）,example 1 | 1,3 |
| Y | | 2 |
| A | | 11,12 |
| Y | CN1887859A（WUHAN YUANDA PHARM GROUP CORP LTD）,03 Jan.2007（03.01.2007）,claims | 2 |
| Y | CN1687027A（XINYI PHARM CO LTD HENAN PROV）,26 Oct. 2005（26.10.2005）,claims | 2 |

☒ Further documents are listed in the continuation of Box C. ☒ See patent family annex.

* Special categories of cited documents:

"A" document defining the general state of the art which is not considered to be of particular relevance

"E" earlier application or patent but published on or after the international filing date

"L" document which may throw doubts on priority claim (S) or which is cited to establish the publication date of another citation or other special reason (as specified)

"O" document referring to an oral disclosure, use, exhibition or other means

"P" document published prior to the international filing date but later than the priority date claimed

"T" later document published after the international filing date or priority date and not in conflict with the application but cited to understand the principle or theory underlying the invention

"X" document of particular relevance; the claimed invention cannot be considered novel or cannot be considered to involve an inventive step when the document is taken alone

"Y" document of particular relevance; the claimed invention cannot be considered to involve an inventive step when the document is combined with one or more other such documents, such combination being obvious to a person skilled in the art

"&"document member of the same patent family

| Date of the actual completion of the international search | Date of mailing of the international search report |
|---|---|
| 05 Mar. 2008(05.03.2008) | **27 Mar. 2008 (27.03.2008)** |

Name and mailing address of the ISA/CN
The State Intellectual Property Office, the P.R.China
6 Xitucheng Rd., Jimen Bridge, Haidian District, Beijing, China
100088
Facsimile No. 86-10-62019451

Authorized officer
Luoling
Telephone No. (86-10)62084127

Form PCT/ISA/210 (second sheet) (April 2007)

**INTERNATIONAL SEARCH REPORT**

International application No.
PCT/CN2007/003859

C (Continuation). DOCUMENTS CONSIDERED TO BE RELEVANT

| Category* | Citation of document, with indication, where appropriate, of the relevant passages | Relevant to claim No. |
|---|---|---|
| X | Wang Dongyang et al., 'A new synthesis of tiopronin', Chinese Journal of Medicinal Chemistry, 7 (1), Mar.1997 (03.1997), pages 55-56 | 4 |
| X | CN1969937A (HUANG Zhenhua), 30 May.2007 (30.05.2007), claims, lines 11-14 of page 1, lines 4-14 of page 4 and example | 5-10 |

Form PCT/ISA/210 (continuation of second sheet) (April 2007)

**INTERNATIONAL SEARCH REPORT**
Information on patent family members

International application No.
PCT/CN2007/003859

| Patent Documents referred in the Report | Publication Date | Patent Family | Publication Date |
|---|---|---|---|
| JP2204478 A | 14.08.1990 | NONE | |
| CN1887859A | 03.01.2007 | NONE | |
| CN1687027A | 26.10.2005 | CN1305845C | 21.03.2007 |
| CN1969937A | 30.05.2007 | NONE | |

Form PCT/ISA/210 (patent family annex) (April 2007)

## INTERNATIONAL SEARCH REPORT

International application No.
PCT/CN2007/003859

Continuation of : CLASSIFICATION OF SUBJECT MATTER

C07C323/58(2006.01)i
C07C319/02(2006.01)i
A61K31/198(2006.01) i
A61P39/02(2006.01) i
A61P43/00(2006.01) i
C07C323/50(2006.01)n
C07C323/22(2006.01)n
C07C323/00(2006.01)n
C07C319/00(2006.01)n

Form PCT/ISA/210 (extra sheet) (April 2007)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- CN 02129300


### Non-patent literature cited in the description


- *Chinese Journal of Medicinal Chemistry,* March 1997, vol. 7 (1), 55-56